# FASCICULE DE BREVET EUROPEEN

(11) **EP 2 955 997 B1**
(45) Date de publication et mention de la délivrance du brevet: **21.11.2018**
(21) Numéro de dépôt: 14702883.1
(22) Date de dépôt: 06.02.2014
(51) Int. Cl.: A01K 11/00

(54) **SYSTÈME D'IDENTIFICATION ANIMALE COMPRENANT UNE PARTIE MÂLE, UNE PARTIE FEMELLE ET UN DISPOSITIF AMOVIBLE DE STOCKAGE D'UN ÉCHANTILLON**
TIERIDENTIFIKATIONSSYSTEM MIT EINEM STECKERTEIL, EINEM BUCHSENTEIL UND EINER ABNEHMBAREN VORRICHTUNG ZUM SPEICHERN EINER PROBE
ANIMAL IDENTIFICATION SYSTEM INCLUDING A MALE PORTION, FEMALE PORTION, AND REMOVABLE DEVICE FOR STORING A SAMPLE

(30) Priorité: 12.02.2013 FR 1351171
(43) Date de publication de la demande: 23.12.2015
(73) Titulaire: Allflex Europe, 35500 Vitré (FR)
(72) Inventeur: DECALUWE, Johan, 53000 Laval (FR); HILPERT, Jean-Jacques, 35500 Vitré (FR); TEYCHENE, Bruno, 81430 Mouzieys-teulet (FR); DE MEULEMEESTER, Johan, 9840 De Pinte (BE); DESTOUMIEUX, Jean-Jacques, 81380 Lescure D'albigeois (FR)
(74) Mandataire: Vidon Brevets & Stratégie
(86) Numéro de dépôt international: PCT/EP2014/052348
(87) Numéro de publication internationale: WO 2014/124863

(56) Documents cités:
- FR-A1- 2 550 915
- FR-A1- 2 917 574
- FR-A1- 2 939 281

## Description

### 1. Domaine de l'invention

Le domaine de l'invention est celui de l'identification et/ou du marquage des animaux.

Plus précisément, l'invention concerne un système d'identification animale permettant d'apposer une marque d'identification visuelle et/ou électronique sur toute espèce animale, et de prélever un échantillon de tissu correspondant.

Un tel prélèvement de tissu, qui peut être effectué simultanément à la pose de la marque d'identification, permet notamment de conserver des cellules/tissus portant des caractéristiques biologiques ou biochimiques de l'animal, par exemple pour identifier ultérieurement l'animal ou détecter des maladies de l'animal.

### 2. Art antérieur

Afin notamment d'améliorer le suivi du cheptel, améliorer la productivité (en éliminant les animaux malades, ou en recherchant des caractéristiques génétiques singulières par exemple), et/ou garantir l'origine des animaux destinés à la consommation (par exemple en détectant des maladies), il est de plus en plus souvent procédé à un ou plusieurs prélèvements de tissu des animaux concernés.

Un tel prélèvement peut être effectué sur l'animal lors de la pose d'une marque d'identification de l'animal (à la naissance par exemple) ou plus tard. D'autres prélèvements peuvent également être effectués tout au long de l'existence de l'animal, par exemple pour détecter des maladies ou certifier l'identité de l'animal, par comparaison des séquences ADN, ou évaluer sa valeur génétique. Une fois collecté, l'échantillon de tissu animal peut donc être stocké et/ou transmis à un laboratoire pour analyse.

Ainsi, parmi les techniques de prélèvement actuellement utilisées, la technique décrite notamment dans la demande de brevet WO02/39810 au nom de CAISLEY permet de prélever un échantillon de tissu animal simultanément à la pose d'une marque d'identification.

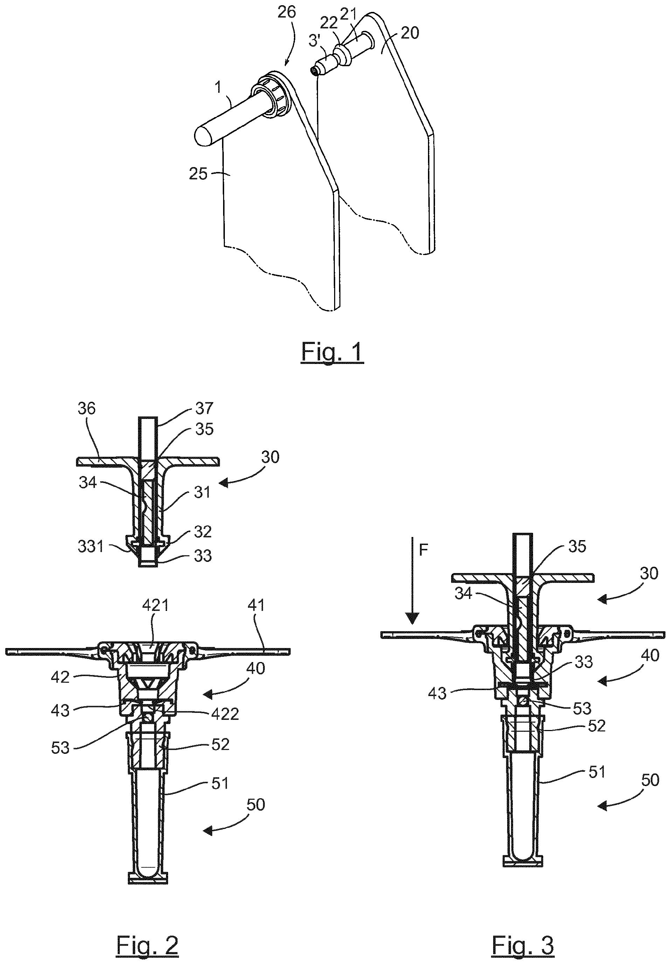

Selon cette technique, illustrée en figure 1, un système d'identification animale comprend une partie mâle 20, une partie femelle 25, et dispositif de stockage d'un échantillon 1. La partie mâle 20 est munie d'un fût mâle 21 présentant une tête 22, à l'extrémité de laquelle est fixée un emporte-pièce 3'. La partie femelle 25 présente un orifice 26 de réception de la tête 22 de la partie mâle, et un canal traversant à l'extrémité duquel est fixé le dispositif de stockage 1, préalablement au prélèvement d'échantillon.

Lors de l'opération de prélèvement et de pose de la marque, l'emporte-pièce 3' vient découper l'oreille de l'animal, traverser le canal traversant la partie femelle 25, et se loger dans le dispositif de stockage 1. Le dispositif de stockage 1, fermé par l'emporte-pièce 3', est alors détaché de la partie femelle 25.

Une telle technique, bien qu'offrant de nombreux avantages puisqu'elle permet le prélèvement de tissus animal simultanément à la pose de la marque, présente également des inconvénients.

En effet, la mise en oeuvre simultanée du prélèvement et de la pose d'une marque d'identification nécessite l'utilisation d'une partie femelle présentant un canal traversant, encore appelée partie femelle à « tête ouverte ».

Or une telle marque (ou boucle) est moins sûre en termes d'inviolabilité, puisque la tête de la partie mâle reste visible et accessible, après emboitement des parties mâle et femelle. Il existe donc un risque accru qu'une personne malveillante repousse la partie mâle hors de la partie femelle, séparant ainsi les parties mâle et femelle, et repose la marque sur un autre animal.

Il existe donc un besoin pour une nouvelle technique permettant d'effectuer simultanément la pose d'une marque d'identification et le prélèvement d'un échantillon de tissu, mais qui ne présente pas ces inconvénients de l'art antérieur. FR-A-2 917 574 décrit un système d'identification comme revendiqué dans la première partie de la revendication 1.

### 3. Exposé de l'invention

L'invention propose une solution nouvelle sous la forme d'un système d'identification animale comprenant :
- une partie mâle, coopérant avec au moins un élément de coupe destiné à découper un échantillon de tissu animal,
- une partie femelle à tête ouverte, présentant un canal traversant, et
- un dispositif amovible de stockage de l'échantillon, destiné à être fixé à une extrémité du canal traversant.

Selon l'invention, un tel système comprend également des moyens de fermeture ou d'obstruction de la tête pouvant prendre au moins deux positions, dont une position d'ouverture permettant le passage de l'échantillon au travers du canal traversant lors d'un prélèvement, et une position de fermeture définitive ou irréversible permettant de fermer le canal au voisinage de l'extrémité postérieurement au prélèvement.

Ainsi, l'invention repose sur une approche nouvelle et inventive du marquage et du prélèvement de tissus animal, permettant d'utiliser une partie femelle à « tête ouverte » pour l'opération de prélèvement, et une partie femelle à « tête fermée » qui reste sur l'animal postérieurement au prélèvement.

Selon l'invention, la tête de la partie femelle peut donc prendre deux états : un état ouvert, antérieur au prélèvement, dans lequel l'échantillon peut traverser la tête de la partie femelle et venir se loger dans le dispositif de stockage, et un état fermé, postérieur au prélèvement. De cette façon, la marque posée sur l'animal correspond à une marque à « tête fermée », considérée comme plus sûre que les marques à tête ouverte. De plus, si l'élément de coupe est un emporte-pièce fixé à l'extrémité du fût mâle, la « fermeture » de la tête de la partie femelle permet de protéger l'arête coupante de l'emporte-pièce une fois la marque posée sur l'animal, et permet donc d'éviter les coupures/blessures des animaux et/ou des éleveurs. En particulier, afin de renforcer l'inviolabilité de la marque ainsi obtenue, la fermeture de la partie femelle est irréversible.

On note que le passage de la position d'ouverture antérieure au prélèvement, dans laquelle le canal est ouvert de part en part pour permettre le passage de l'échantillon au travers du canal traversant lors du prélèvement, à la position de fermeture irréversible postérieure au prélèvement, dans laquelle le canal est fermé au voisinage de son extrémité à laquelle était fixé le dispositif amovible de stockage, peut être commandé par l'opération de prélèvement. Par exemple, les moyens de fermeture peuvent être commandés par le retrait du dispositif amovible de stockage et/ou par le déplacement d'une pièce dans le système, comme le coulissement du bouchon de la partie mâle dans la partie femelle et/ou par un outil de pose et de prélèvement.

Selon un premier mode de réalisation, les moyens de fermeture comprennent un bouchon.

Un tel bouchon peut être inséré manuellement dans le canal traversant de la partie femelle, au voisinage de l'extrémité à laquelle était fixé le dispositif de stockage, ou de façon automatique ou quasi-automatique, c'est-à-dire sans action particulière de l'utilisateur, postérieurement au prélèvement.

En particulier, les moyens de fermeture comprennent des moyens de retenue du bouchon dans la position de fermeture.

Ces moyens de retenue, localisés à l'intérieur de la partie femelle et solidaires de la partie femelle, ne sont pas visibles ou accessibles. Ils permettent donc de maintenir le bouchon de façon sûre et définitive.

Selon un premier exemple de réalisation, les moyens de retenue comprennent une rondelle présentant au moins une lame ressort ou griffe.

Il s'agit par exemple d'une rondelle d'arrêt de type « Starlock » (marque déposée).

Selon un deuxième exemple de réalisation, les moyens de retenue comprennent au moins un pêne pouvant prendre au moins deux positions, dont une position de déverrouillage dans laquelle le ou les pênes sont maintenus en position par le dispositif de stockage, et une position de verrouillage dans laquelle le ou les pênes sont libérés et retiennent le bouchon.

En particulier, la position de verrouillage est obtenue lorsque le dispositif de stockage est désolidarisé de la partie femelle. Ainsi, si une personne malveillante tente de remplacer le dispositif de stockage initial par un autre (qui porte un autre numéro d'identifiant par exemple), la tête se ferme lorsque le dispositif de stockage initial est désolidarisé de la partie femelle, et il n'est plus possible d'y fixer un autre dispositif de stockage. Il est donc impossible d'échanger les dispositifs de stockage de l'échantillon.

Selon un aspect particulier de ce deuxième exemple de réalisation, les moyens de retenue comprennent également au moins un élément de blocage (de type bille ou cylindre par exemple) permettant de bloquer le ou les pênes dans la position de verrouillage.

Selon un troisième exemple de réalisation, les moyens de retenue comprennent une pièce formant ressort, comprenant au moins deux mâchoires mobiles telles que dans ladite position d'ouverture, lesdites mâchoires sont contraintes en ouverture et maintiennent ledit dispositif de stockage, et dans ladite position de fermeture, lesdites mâchoires sont libérées et verrouillent ledit bouchon.

Selon ce troisième exemple de réalisation, le passage de la position d'ouverture à la position de fermeture peut être activé par le retrait du dispositif de stockage.

Selon une caractéristique particulière de ces exemples de réalisation, le bouchon comprend au moins une gorge, destinée à recevoir une extrémité libre de la ou des lames ressort, du ou des pênes, ou des mâchoires, dans la position de fermeture.

Selon un quatrième exemple de réalisation, le bouchon présente un premier diamètre dans la position d'ouverture, et un deuxième diamètre, supérieur au premier diamètre, dans la position de fermeture.

Un tel bouchon, qui présente une forme de révolution (cylindrique ou sphérique) peut donc être expansif et augmenter de volume lorsqu'il se trouve au voisinage de l'extrémité de la partie femelle à boucher.

Selon un cinquième exemple de réalisation, le bouchon comprend une substance spécifique, libérée dans la position de fermeture.

Une telle substance spécifique permet par exemple une réaction chimique entre ladite substance et la matière de la partie femelle (ou d'un élément de la partie femelle), comme une polymérisation, permettant de « souder » le bouchon à l'intérieur de la partie femelle. Il peut notamment s'agir d'une colle ou d'une résine.

En particulier, le bouchon peut être présent dans un fût de la partie mâle dans la position d'ouverture.

Ainsi, le bouchon est prévu dans la partie mâle, et peut venir s'insérer automatiquement ou quasi-automatiquement dans les moyens de retenue de la partie femelle postérieurement au prélèvement.

Selon un deuxième mode de réalisation, les moyens de fermeture comprennent au moins un obturateur mobile entre deux positions, dont la position d'ouverture ou de déverrouillage dans laquelle le ou les obturateurs sont maintenus en position par le dispositif de stockage, et la position de fermeture dans laquelle le ou les obturateurs obturent le canal au voisinage de l'extrémité à laquelle était fixée le dispositif de stockage.

Selon ce deuxième mode de réalisation, le passage de la position d'ouverture à la position de fermeture peut être activé par le retrait du dispositif de stockage. En d'autres termes, le ou les obturateurs sont libérés lorsque le dispositif de stockage est désolidarisé/retiré de la partie femelle.

Ainsi, plutôt qu'introduire un bouchon à l'intérieur du canal traversant de la partie femelle, un ou plusieurs obturateurs peuvent être prévus pour obturer l'extrémité du canal à laquelle peut être fixé le dispositif de stockage. Ces moyens de fermeture sont solidaires de la partie femelle.

Par exemple, ces obturateurs peuvent prendre la forme de volets ou de mâchoires.

Ainsi, les moyens de fermeture comprennent par exemple une pièce formant ressort comprenant au moins deux mâchoires mobiles telles que dans ladite position d'ouverture, lesdites mâchoires sont contraintes en ouverture et maintiennent ledit dispositif de stockage, et dans ladite position de fermeture, lesdites mâchoires sont libérées et obturent le canal au voisinage de l'extrémité à laquelle était fixée le dispositif de stockage.

Selon une autre caractéristique particulière, le système d'identification animale comprend un élément de renfort, inséré dans le fût de la partie mâle, encore appelé raidisseur, destiné à renforcer la rigidité du fût lors du prélèvement.

Ainsi, plutôt qu'utiliser un fût mâle rigide, qui risquerait de casser si la marque d'identification de l'animal est coincée dans un obstacle (clôture, barrière, branche, etc), ce qui conduirait à la perte de la marque, il est possible d'utiliser une matière « souple » ou « flexible », de type polyuréthane par exemple, déformable, permettant notamment à l'animal de se dégager d'un tel obstacle.

Un tel élément de renfort est par exemple un tube de renfort amovible, inséré dans le fût mâle pour renforcer la rigidité du fût lors de l'opération de prélèvement/pose de la marque. Une fois la marque posée, le tube de renfort est retiré du fût mâle, qui peut désormais se déformer.

Selon une variante, l'élément de renfort est un ressort à spires jointives. Un tel ressort permet la transmission d'un effort à l'élément de coupe lors de l'opération de prélèvement/pose de la marque. De plus, il résiste en compression, ce qui évite au fût mâle de se comprimer. Une fois la marque posée, un tel ressort permet la flexion/déformation du fût mâle, notamment lorsque le fût mâle est réalisé avec une matière « souple » ou « flexible ».

Un tel élément de renfort sert également de guide au bouchon, lorsqu'il est présent dans la partie mâle dans la position d'ouverture.

Selon un autre aspect de l'invention, la partie mâle, la partie femelle et le dispositif de prélèvement portent chacun un identifiant permettant d'identifier l'animal.

Ces trois éléments peuvent porter un même numéro d'identification de l'animal, ou des identifiants liés entre eux. Dans tous les cas, ces identifiants permettent de coder la même information, c'est-à-dire d'identifier le même animal.

De cette façon, on s'assure que l'animal et l'échantillon prélevé sont correctement identifiés et liés.

Encore un autre aspect de l'invention concerne un système d'identification comprenant également un élément poussoir mobile par rapport à l'élément de coupe, permettant de pousser l'échantillon dans le dispositif de stockage et d'obturer le dispositif de stockage.

Selon cet aspect, l'invention permet un prélèvement optimisé, particulièrement simple et rapide pour l'utilisateur. L'utilisation de deux éléments distincts, l'un pour découper les tissus et l'autre pour pousser l'échantillon, présente de nombreux avantages. Par exemple, le fait de découper les tissus puis de pousser l'échantillon dans le dispositif de stockage permet d'assurer une bonne découpe de l'échantillon, et d'éviter que des poils restent coincés entre les parois et le bouchon du dispositif de stockage. Ces deux éléments distincts permettent également d'obtenir un meilleur prélèvement. En effet, l'élément de coupe n'étant pas destiné à être inséré dans le dispositif de stockage, il est possible d'augmenter la taille de l'élément de coupe, c'est-à-dire la longueur de son arête coupante, et donc d'augmenter la taille de l'échantillon prélevé. De plus, on évite une contamination potentielle de l'échantillon, puisque l'utilisateur n'a pas à agir directement sur l'échantillon. Par ailleurs, l'échantillon étant découpé par l'élément de coupe, puis automatiquement poussé dans le dispositif de stockage par un élément poussoir, aucune portion de l'outil ou aucun élément extérieur n'est en contact direct avec le tissu prélevé.

En particulier, le dispositif de stockage comprend un tube et une tête de tube, la tête de tube formant un support configuré pour servir de surface d'appui à l'élément de coupe pour découper l'échantillon, et comprenant une ouverture permettant l'insertion d'au moins une portion de l'élément poussoir dans le dispositif de stockage.

L'utilisation d'une tête de tube procure de nombreux avantages. Tout d'abord, elle peut procurer un support sur lequel l'élément de coupe peut s'appuyer pour découper correctement les tissus de l'animal. Elle permet également la fermeture du tube, par exemple par emboîtement ou clippage de l'élément poussoir dans la tête de tube. De plus, la présence d'une telle tête de tube permet l'automatisation de l'ouverture des tubes par les laboratoires d'analyse, en décapsulant la tête de tube de façon que seul l'échantillon reste à l'intérieur du tube.

En particulier, préalablement au prélèvement, le tube est fermé par un élément lesté de type sphère ou cylindre.

Ainsi, dans une première position, l'élément lesté est maintenu dans la partie supérieure du dispositif de stockage et obture un orifice d'entrée du tube. Dans cette première position préalable au prélèvement, correspondant par exemple à la forme sous laquelle les tubes sont commercialisés, l'élément lesté remplit la fonction de bouchon du tube, permettant d'éviter l'introduction d'impuretés dans le tube, et donc de contaminer l'intérieur du tube par l'environnement extérieur.

Dans la deuxième position postérieure au prélèvement, l'élément lesté est libéré dans le tube. Il peut alors remplir la fonction d'agitateur ou mélangeur. Il peut également servir de lest, permettant de faire couler l'échantillon au fond du tube, de « pilon », ou encore d'indicateur visuel d'un prélèvement réussi.

### 4. Liste des figures

D'autres caractéristiques et avantages de l'invention apparaîtront plus clairement à la lecture de la description suivante d'un mode de réalisation particulier, donné à titre de simple exemple illustratif et non limitatif, et des dessins annexés, parmi lesquels :
- la figure 1 présente un système d'identification animale selon l'art antérieur ;
- les figures 2 à 5 présentent un système d'identification animale selon un mode de réalisation particulier de l'invention, à différents stades du marquage/prélèvement ;
- les figures 6, 7, 10 à 12 illustrent un exemple de mise en oeuvre des moyens de retenue d'un bouchon selon un premier mode de réalisation de l'invention ;
- les figures 8, 9, 13 et 14 illustrent un exemple de mise en oeuvre des moyens de fermeture selon un deuxième mode de réalisation de l'invention.

### 5. Description d'un mode de réalisation de l'invention

L'invention concerne un système d'identification animale permettant le prélèvement de tissu animal conjointement à la pose d'une marque d'identification, et renforçant l'inviolabilité de la marque posée.

Pour ce faire, le principe général de l'invention repose sur l'utilisation d'une partie femelle pouvant prendre deux états, dont un état « tête ouverte » antérieur au prélèvement, et un état « tête fermée » postérieur au prélèvement.

Plus précisément, un système d'identification animale selon l'invention comprend :
- une partie mâle, coopérant avec au moins un élément de coupe destiné à découper un échantillon de tissu animal,
- une partie femelle à tête ouverte, présentant un canal traversant, c'est-à-dire ouvert de part en part, et
- un dispositif amovible de stockage de l'échantillon, destiné à être fixé à une extrémité du canal traversant.

Le système comprend également des moyens de fermeture de la tête pouvant prendre au moins deux positions, dont une position d'ouverture permettant le passage de l'échantillon au travers du canal lors d'un prélèvement, et une position de fermeture permettant de fermer le canal au voisinage de l'extrémité à laquelle peut être fixé le dispositif de stockage postérieurement au prélèvement.

On décrit ci-après un premier mode de réalisation de l'invention, dans lequel les moyens de fermeture de la tête de la partie femelle comprennent un bouchon.

Plus précisément, comme illustré en figure 2, on considère un système d'identification animale comprenant une partie mâle 30, une partie femelle 40 et un dispositif de stockage 50.

La partie mâle 30 est munie d'un fût mâle 31 s'étendant à partir d'une base 36, qui peut servir de support de marquage. Cette base peut prendre la forme d'un disque, d'un carré, d'un rectangle, d'une étiquette, etc. Le fût mâle présente à son extrémité une tête 32 en forme de pointe tronquée. Un élément de coupe 33, également appelé emporte-pièce, est fixé à l'extrémité de la pointe tronquée 32, et présente une arête coupante permettant de découper les tissus de l'animal. Eventuellement, l'élément de coupe comprend un support 331 (par exemple en plastique rigide), surmoulé lors de la fabrication de la partie mâle de façon à solidariser l'élément de coupe au fût mâle. Selon une autre variante de réalisation, l'élément de coupe prend la forme d'une aiguille de biopsie, qui coulisse à l'intérieur du fût mâle pour découper les tissus de l'animal.

Un tel élément de coupe 33 est ouvert à ses deux extrémités, afin de laisser passer d'une part un élément poussoir 34, permettant de pousser l'échantillon de tissus découpés dans le dispositif de stockage, et d'autre part un bouchon 35, permettant de fermer la tête de la partie femelle.

L'élément poussoir 34 et le bouchon 35 sont donc mobiles par rapport à l'élément de coupe 33, et peuvent coulisser au travers de l'élément de coupe 33 pour :
- pousser l'échantillon de tissu et fermer le dispositif de stockage 50 pour l'élément poussoir 34, et
- pousser l'élément poussoir 34 et fermer la tête 42 de la partie femelle pour le bouchon 35.

L'élément poussoir 34 et le bouchon 35 présentent avantageusement une forme de révolution et un diamètre légèrement inférieur à celui de l'élément de coupe afin de pouvoir glisser à l'intérieur du cylindre formant l'élément de coupe.

On note que l'utilisation d'un élément poussoir est notamment décrite dans la demande WO2010/066475 au nom du même Demandeur et n'est pas décrite plus en détails.

Bien entendu, l'utilisation d'un tel élément poussoir est facultative, et correspond uniquement à un mode de réalisation particulier de l'invention.

Finalement, un élément de renfort 37, encore appelé raidisseur, peut être inséré dans le fût 31 de la partie mâle, pour renforcer la rigidité du fût mâle lors du marquage et/ou du prélèvement, notamment lorsque la partie mâle est réalisée avec une matière plastique souple, comme le polyuréthane. Un tel élément de renfort est par exemple un tube de renfort amovible inséré dans le fût mâle préalablement au prélèvement et/ou solidarisé à l'outil de prélèvement, puis retiré postérieurement au prélèvement. Avantageusement, il reste solidarisé à l'outil de prélèvement postérieurement au prélèvement, et peut être éjecté de l'outil de façon classique (en appuyant sur une gâchette par exemple). Selon une variante, l'élément de renfort est un ressort à spires jointives, résistant en compression ce qui évite au fût mâle de se comprimer. Une fois la marque posée, un tel ressort permet la flexion/déformation du fût mâle.

Un tel raidisseur prend par exemple la forme d'un cylindre creux, ouvert à ses deux extrémités, à l'intérieur duquel coulissent l'élément poussoir 34 et le bouchon 35. Il peut s'étendre sur toute la longueur du fût mâle, à partir de la base de l'élément de coupe (extrémité non coupante). Il peut ainsi transmettre un effort à l'élément de coupe, lors des opérations de marquage et de prélèvement. De plus, la mise en place d'un raidisseur permet l'utilisation d'un élément de coupe de faible longueur.

La partie femelle 40 comprend quant à elle une base 41 pouvant servir de support de marquage. Comme pour la partie mâle, cette base peut prendre différente forme (disque, carré, rectangle, étiquette, etc).

La partie femelle 40 présente par ailleurs, à l'état initial, une tête ouverte 42. Une telle tête 42 comprend un canal traversant, c'est-à-dire ouvert de part en part, permettant de recevoir au moins partiellement le fût 31 de la partie mâle, introduit par une première extrémité 421 du canal. Un dispositif de stockage 50 peut être fixé à l'autre extrémité 422 du canal.

Selon ce premier mode de réalisation, la partie femelle 40 comprend également des moyens de retenue 43, permettant de retenir le bouchon 35 dans la partie femelle dans la position de fermeture, de façon à fermer le canal au voisinage de l'extrémité 422 à laquelle peut être fixée le dispositif de stockage 50.

Finalement, le dispositif de stockage 50 comprend, selon ce premier mode de réalisation, un tube 51 destiné à recevoir l'échantillon, muni d'une tête de tube 52 et d'un élément lesté 53, par exemple une bille.

Un tel dispositif de stockage peut être fixé par tout moyen à l'extrémité 422 de la tête de la partie femelle. Par exemple, un tel dispositif peut être vissé dans le canal traversant de la partie femelle, emboité à force dans la partie femelle, clippé à la partie femelle, etc.

Selon cet exemple, la tête de tube 52 est solidarisée à l'entrée du tube 51, par exemple par clippage, emboîtement, etc. Elle peut être réalisée en matière flexible, notamment en caoutchouc, pour faciliter son insertion dans le col du tube. Plus précisément, la tête de tube 52 se présente sous la forme d'un capuchon percé d'une ouverture centrale, de diamètre suffisant pour permettre l'insertion de la bille 53. La tête de tube 52 présente également une collerette, pouvant prendre appui sur le rebord du tube 51. L'utilisation d'une telle collerette permet notamment de faciliter la pose et l'enlèvement du capuchon. La collerette définit également une surface de butée sur laquelle l'arête coupante d'un élément de coupe peut prendre appui lors du prélèvement, afin de découper plus facilement l'échantillon de tissus.

Ainsi, dans une première position préalable au prélèvement de l'échantillon, la bille 53 est maintenue en position dans le dispositif de stockage, et obture l'orifice d'entrée du tube 51 pour assurer une fonction de bouchon du tube. Dans une deuxième position, ultérieure au prélèvement de l'échantillon, la bille est introduite à l'intérieur du tube en même temps que l'échantillon, et libérée à l'intérieur du tube. Elle peut donc assurer une fonction d'agitateur ou mélangeur, de lest, de pilon, ou encore d'indicateur visuel d'un prélèvement réussi.

Bien entendu, l'utilisation d'une tête de tube et/ou d'un élément lesté de type bille est facultative, et correspond uniquement à un mode de réalisation particulier de l'invention.

On considère finalement que la forme du tube de réception 51 est compatible avec un support de tubes d'échantillon de type microplaque, comprenant par exemple 24, 48 ou 96 positions.

Selon l'invention, comme illustré en figure 2, la partie femelle 40 présente une tête ouverte (c'est-à-dire un canal traversant) préalablement aux opérations de marquage et de prélèvement. Les parties mâle 30 et femelle 40, et le dispositif de stockage 50, dans cet état initial, peuvent être montés sur un outil de pose.

Par souci de simplification, l'outil de pose n'est pas représenté sur les figures. Il s'agit d'un outil fonctionnant en deux temps, permettant de déplacer simultanément l'élément de coupe et le bouchon (et éventuellement l'élément poussoir qui, on le rappelle, est facultatif) dans un premier temps, puis de déplacer uniquement le bouchon (et éventuellement l'élément poussoir) dans un second temps. Un tel outil est notamment décrit dans la demande de brevet WO2011154510 au nom du même Demandeur.

Comme illustré en figure 3, au cours des opérations de marquage et de prélèvement, les parties mâle et femelle sont emboîtées. Plus précisément, un premier mouvement de translation permet d'amener l'élément de coupe 33 au contact des tissus de l'animal, de découper les tissus, et d'introduire au moins partiellement le fût 31 de la partie mâle dans le canal traversant de la partie femelle.

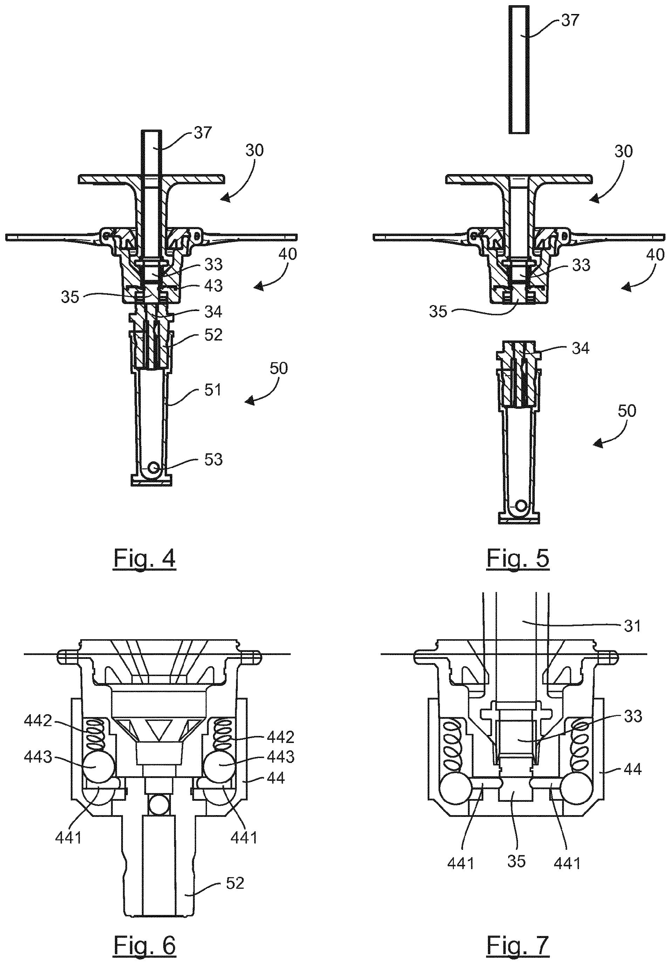

Lorsque l'élément de coupe 33 arrive en butée contre la tête de tube 52 (éventuellement par l'intermédiaire des moyens de retenue 43), un deuxième mouvement de translation, selon le même axe de translation que le premier mouvement, est déclenché, permettant de pousser l'élément poussoir 34 et le bouchon 35 au travers de l'élément de coupe 33, selon la direction illustrée par la flèche F, comme illustré en figure 4.

Plus précisément, l'élément poussoir 34 coulisse à travers l'élément de coupe 33, pousse l'échantillon de tissus et la bille 53 dans le tube 51, et vient s'emboiter dans la tête du tube 52, obturant ainsi hermétiquement ou quasi-hermétiquement le tube 51.

De façon similaire, le bouchon 35 pousse l'élément de coupe 34, coulisse à travers l'élément de coupe 33, et arrête sa course lorsqu'il est retenu par les moyens de retenue 43, obturant ainsi l'extrémité 422 du canal traversant de la tête 42 de la partie femelle.

Les opérations de prélèvement et de marquage sont alors effectuées.

Comme illustré en figure 5, la marque d'identification formée par l'emboitement de la partie mâle 30 et la partie femelle 40 reste sur l'animal (au niveau de son oreille par exemple), et l'élément de renfort 37 et le dispositif de stockage 50, contenant l'échantillon prélevé, restent maintenus sur l'outil de pose. Ils peuvent alors être éjectés ou retirés de façon classique.

On décrit ci-après plus en détail les moyens de fermeture de la tête de la partie femelle selon ce premier mode de réalisation mettant en oeuvre un bouchon 35.

Selon un premier exemple, on considère que les moyens de retenue 43, présents dans la partie femelle, comprennent une rondelle présentant au moins une lame ressort ou griffe. Il s'agit par exemple d'une rondelle d'arrêt de type « Starlock » (marque déposée), qui peut être surmoulée à l'intérieur de la tête de la partie femelle ou assemblée avec la tête de la partie femelle.

Une telle rondelle est positionnée au voisinage de l'extrémité 422 du canal à laquelle le dispositif de stockage peut être fixé. L'axe principal de la rondelle et celui du canal de la partie femelle coïncident. De ce fait, la rondelle n'empêche pas le passage de l'échantillon de tissus et de l'élément poussoir 34. En revanche, la rondelle retient le bouchon 35.

Par exemple, le bouchon 35 comprend une ou plusieurs gorges ou rainures, à l'intérieur desquelles viennent s'insérer la ou les lames ressort pour retenir le bouchon 35. De cette façon, le bouchon 35 se trouve bloqué au voisinage de l'extrémité 422 du canal à laquelle le dispositif de stockage peut être fixé, et la tête de la partie femelle est dans un état fermé. Ces moyens de retenue permettent de maintenir le bouchon de façon sûre et irréversible.

Selon un deuxième exemple, illustré en figures 6 et 7, les moyens de retenue comprennent un ou plusieurs pênes 441 (par exemple trois) pouvant prendre au moins deux positions, dont une position de déverrouillage, illustrée en figure 6, dans laquelle le ou les pênes 441 sont maintenus en position par le dispositif de stockage, et une position de verrouillage, illustrée en figure 7, dans laquelle le ou les pênes 441 sont libérés et retiennent le bouchon 35.

Plus précisément, la partie femelle 40 comprend, selon ce deuxième exemple, un chapeau 44, recouvrant partiellement la tête 42 de la partie femelle et le dispositif de stockage 50, au voisinage de l'extrémité 422 du canal à laquelle peut être fixé le dispositif de stockage.

Préalablement au prélèvement, et pendant le prélèvement, le dispositif de stockage 50 est fixé à la partie femelle 40. Par exemple, la tête de tube 52 est emboitée dans le chapeau 44. Le ou les pênes 441 sont maintenus dans une position de déverrouillage par la tête de tube 52. Une force est également exercée sur le ou les pênes 441, par l'intermédiaire d'un ou plusieurs éléments de rappel 442, de type ressort en compression par exemple, et d'un ou plusieurs éléments de blocage 443 (par exemple trois), de type bille par exemple, pour les maintenir en position.

Postérieurement au prélèvement, le dispositif de stockage 50 est désolidarisé de la partie femelle 40. Le ou les pênes sont alors libérés, et coulissent le long de la tête 42, au voisinage de l'extrémité 422 du canal traversant à laquelle était précédemment fixée la tête de tube 52, pour venir enserrer le bouchon 35 dans la position de verrouillage. Par exemple, si le bouchon 35 présente au moins une gorge ou rainure, le ou les pênes coulissent pour s'engager dans la ou les gorges, de façon à retenir le bouchon 35 au voisinage de l'extrémité 422.

Le coulissement des pênes 441 libère un espace pour les éléments de blocage 443. Sous l'effet des éléments de rappel 442, qui se détendent, les éléments de blocage 443 sont poussés dans ce logement, et bloquent ainsi le ou les pênes en position de verrouillage. De cette façon, le bouchon 35 se trouve bloqué au voisinage de l'extrémité 422 du canal à laquelle le dispositif de stockage peut être fixé, et la tête de la partie femelle est dans un état fermé.

Comme précédemment, ces moyens de retenue permettent de maintenir le bouchon 35 de façon sûre et irréversible.

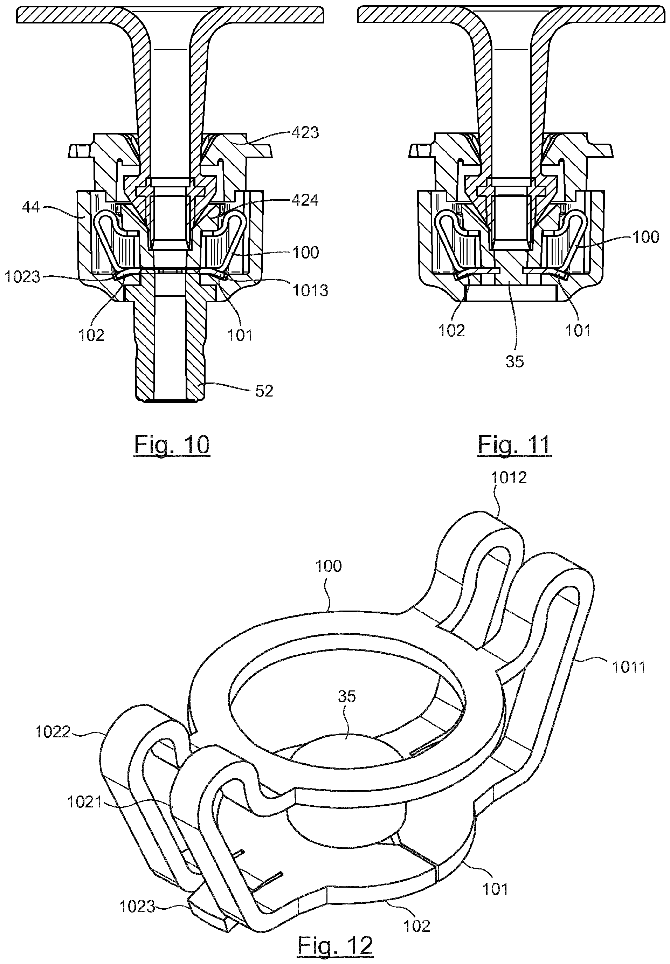

Selon un troisième exemple, illustré en figures 10 à 12, les moyens de retenue comprennent une pièce 100 formant ressort, comprenant au moins deux mâchoires mobiles 101, 102 telles que dans la position d'ouverture, illustrée en figure 10, les mâchoires 101 et 102 sont contraintes en ouverture et maintiennent le dispositif de stockage au niveau de la tête de tube 52, et telles que dans la position de fermeture, illustrée en figure 11, les mâchoires 101 et 102 sont libérées et verrouillent le bouchon 35.

On note que le passage de la position d'ouverture à la position de fermeture peut être activé par le retrait du dispositif de stockage.

Pour ce faire, chacune des mâchoires peut être associée à au moins une lame ressort, tendant à fermer les deux mâchoires 101 et 102. Par exemple, la mâchoire 101 est reliée à deux lames ressorts 1011 et 1012 permettant son écartement dans la position d'ouverture et son rappel dans la position de fermeture. La mâchoire 102 est quant à elle reliée à deux lames ressorts 1021 et 1022 permettant son écartement dans la position d'ouverture et son rappel dans la position de fermeture.

La partie femelle comprend également, selon ce troisième exemple, un chapeau 44, recouvrant partiellement la tête de la partie femelle, et le dispositif de stockage, au voisinage de l'extrémité du canal à laquelle peut être fixé le dispositif de stockage. On note que selon ce mode de réalisation, la tête de la partie femelle est composée d'un anneau 423 permettant de retenir la tête de la partie mâle dans la partie femelle, et d'un élément de centrage 424 qui permet d'assurer un placement correct de la tête de la partie mâle dans la partie femelle, en vu du prélèvement.

Préalablement au prélèvement, et pendant le prélèvement, le dispositif de stockage est fixé à la partie femelle. Par exemple, la tête de tube 52 est maintenue dans le chapeau 44 par les mâchoires 101 et 102 qui viennent l'enserrer. On rappelle que dans cette première position d'ouverture, les mâchoires sont contraintes en ouverture.

Postérieurement au prélèvement, le dispositif de stockage est désolidarisé de la partie femelle. La contrainte appliquée sur les mâchoires 101 et 102 est relâchée, et les mâchoires se ferment au voisinage de l'extrémité du canal traversant à laquelle était précédemment fixée la tête de tube 52, pour venir enserrer le bouchon 35 dans la position de fermeture. Par exemple, si le bouchon 35 présente au moins une gorge ou rainure, les mâchoires peuvent s'engager dans la ou les gorges, de façon à retenir le bouchon 35 au voisinage de l'extrémité 422.

Selon une variante avantageuse, des ergots 1013 et 1023 sont respectivement prévus entre les deux lames ressorts 1011 et 1012 reliées à la mâchoire 101, et entre les deux lames ressorts 1021 et 1022 reliées à la mâchoire 102. De tels ergots 1013 et 1023 permettent notamment d'empêcher le retour des mâchoires 101 et 102 dans la position d'ouverture, ce qui permet de sécuriser le verrouillage du bouchon 35.

Par ailleurs, selon une autre variante avantageuse, les deux mâchoires 101 et 102 présentent une forme adaptée pour coopérer avec la tête de tube 52 dans la position d'ouverture et pour coopérer avec le bouchon 35 dans la position de fermeture.

Comme précédemment, ces moyens de retenue permettent de maintenir le bouchon 35 de façon sûre et irréversible.

Selon un quatrième exemple, non illustré, les moyens de fermeture comprennent un bouchon présentant un premier diamètre dans la position d'ouverture, et un deuxième diamètre, supérieur au premier diamètre, dans la position de fermeture.

Un tel bouchon peut donc être expansif et augmenter de volume lorsqu'il se trouve au voisinage de l'extrémité de la partie femelle à boucher. Par exemple, le bouchon présente une structure alvéolaire qui est compressée dans la position d'ouverture, lorsque le bouchon est présent dans le fût mâle par exemple. Lorsque le bouchon pénètre le canal de la partie femelle, qui présente un diamètre plus large que le diamètre intérieur du fût mâle, la structure alvéolaire peut reprendre sa forme d'origine, et obturer ainsi le canal.

Selon un cinquième exemple, non illustré, le bouchon comprend une substance spécifique, libérée dans la position de fermeture.

Une telle substance spécifique permet par exemple une réaction avec la matière du canal de la partie femelle, comme une polymérisation, permettant de « souder » le bouchon à l'intérieur de la partie femelle. En particulier, une telle substance spécifique peut être une colle ou une résine.

Elle peut être libérée par perforation du bouchon. A cet effet, un élément perforateur peut être prévu dans le canal traversant de la partie femelle.

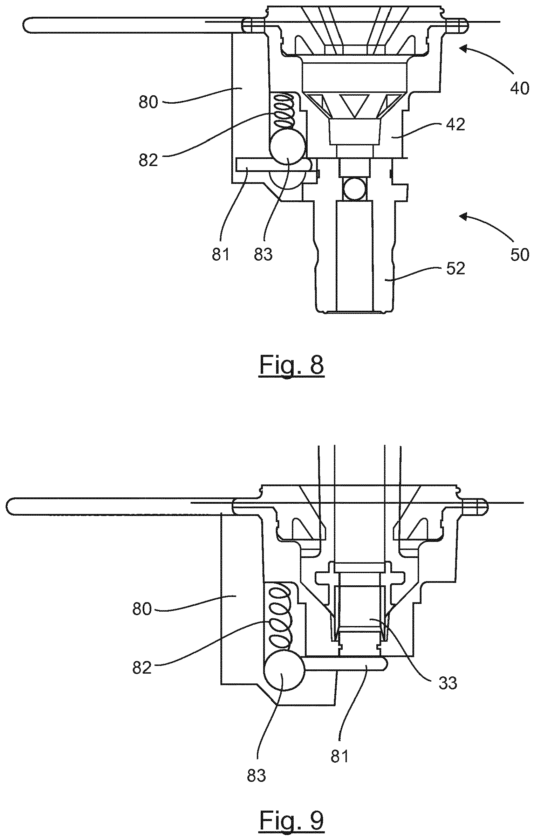

Les figures 8 et 9 illustrent un deuxième mode de réalisation de l'invention, dans lequel les moyens de fermeture comprennent au moins un obturateur 81 mobile entre deux positions, dont la position d'ouverture ou de déverrouillage, illustrée en figure 8, dans laquelle l'obturateur est maintenu en position par le dispositif de stockage, et la position de fermeture ou de verrouillage, illustrée en figure 9 dans laquelle l'obturateur est libéré lorsque le dispositif de stockage est désolidarisé de la partie femelle, et vient obturer le canal au voisinage de l'extrémité à laquelle était fixée le dispositif de stockage.

Plus précisément, la partie femelle 40 comprend, selon ce deuxième mode de réalisation, un chapeau 80, recouvrant partiellement la tête 42 de la partie femelle et le dispositif de stockage 50.

Préalablement au prélèvement, et pendant le prélèvement, le dispositif de stockage 50 est fixé à la partie femelle 40. L'obturateur ou volet 81 est maintenu dans une position d'ouverture ou de déverrouillage par la tête de tube 52. Une force est également exercée sur l'obturateur 81, par l'intermédiaire d'un ou plusieurs éléments de rappel 82, de type ressort en compression par exemple, et d'un ou plusieurs éléments de blocage 83, de type bille par exemple, pour le maintenir en position.

Postérieurement au prélèvement, le dispositif de stockage 50 est désolidarisé de la partie femelle 40. L'obturateur coulisse alors le long de la tête 42, au voisinage de l'extrémité 422 du canal traversant à laquelle était précédemment fixée la tête de tube 52, pour venir obturer l'extrémité 422 du canal dans la position de fermeture ou de verrouillage.

Le coulissement de l'obturateur 81 libère un espace pour l'élément de blocage 83. Sous l'effet de l'élément de rappel 82, qui se détend, l'élément de blocage 83 est poussé dans ce logement, et bloque ainsi l'obturateur 81 en position de verrouillage. De cette façon, l'obturateur ferme l'extrémité 422 du canal de façon sûre et irréversible, et la tête de la partie femelle est dans un état fermé.

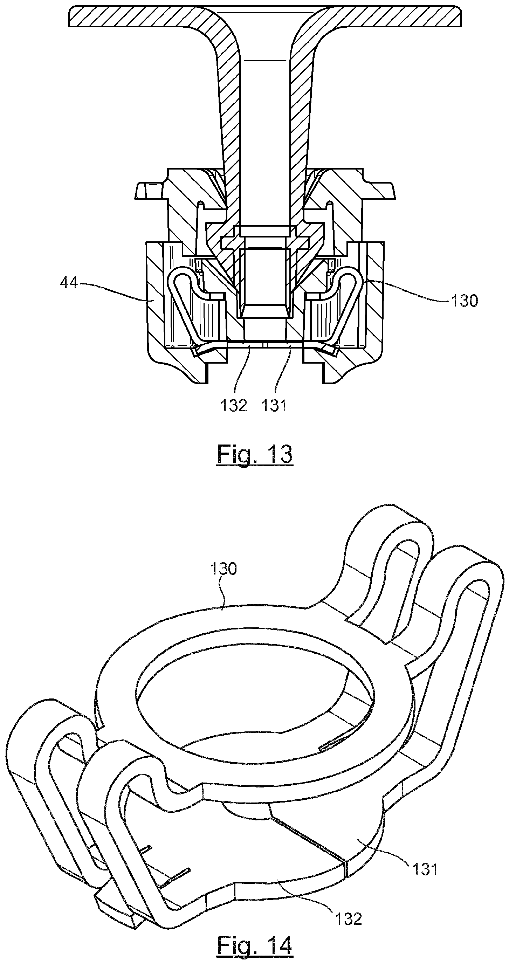

Les figures 13 et 14 illustrent un autre exemple de ce deuxième mode de réalisation de l'invention, dans lequel les moyens de fermeture comprennent une pièce 130 formant ressort comprenant deux obturateurs mobiles, appelés mâchoires 131 et 132, tels que dans la position d'ouverture (non illustrée, mais similaire à la position illustrée en figure 10), les mâchoires 131 et 132 sont contraintes en ouverture et maintiennent le dispositif de stockage au niveau de la tête de tube, et dans la position de fermeture, illustrée en figure 13, les mâchoires 131 et 132 sont libérées et obturent (au moins partiellement) le canal au voisinage de l'extrémité à laquelle était fixée le dispositif de stockage.

La pièce 130 formant ressort est similaire à la pièce 100 décrite en relation avec les figures 10 à 12. Son fonctionnement est donc identique, et n'est pas repris en détail. On note seulement que selon ce deuxième mode de réalisation, les deux mâchoires 131 et 132 présentent avantageusement une forme adaptée pour coopérer avec la tête de tube 52 dans la position d'ouverture, et pour fermer le canal au voisinage de l'extrémité à laquelle était fixée le dispositif de stockage dans la position de fermeture.

De la même façon, la partie femelle comprend également, selon cet exemple, un chapeau 44, tel que décrit en relation avec les figures 10 à 12.

Préalablement au prélèvement, et pendant le prélèvement, le dispositif de stockage est fixé à la partie femelle. Par exemple, la tête de tube est maintenue dans le chapeau 44 par les mâchoires 131 et 132 qui viennent l'enserrer. On rappelle que dans cette première position d'ouverture, les mâchoires sont contraintes en ouverture.

Postérieurement au prélèvement, le dispositif de stockage est désolidarisé de la partie femelle. La contrainte appliquée sur les mâchoires 131 et 132 est relâchée, et les mâchoires se ferment au voisinage de l'extrémité du canal traversant à laquelle était précédemment fixée la tête de tube.

D'autres modes de réalisation sont bien entendu envisageables, mettant en oeuvre un bouchon, un obturateur, ou un autre élément pour fermer la tête de la partie femelle.

En particulier, bien que les modes de réalisation décrits en relation avec les figures 2 à 5 prévoient la présence d'un élément poussoir et d'un élément de renfort, on rappelle que ces éléments sont facultatifs. Le fonctionnement de l'invention, c'est-à-dire la fermeture de la tête, est similaire que le système d'identification animale utilise ou non ces éléments. En particulier, si l'on n'utilise pas d'élément de renfort, il est possible d'utiliser un fût mâle réalisé en matière dure, ou d'utiliser un pointeau de l'outil de pose ou une aiguille de biopsie pour rigidifier le fût mâle. Si l'on n'utilise pas d'élément poussoir, il est possible de prévoir que l'élément de coupe se détache de la partie mâle postérieurement au prélèvement et vienne fermer le tube, ou de prévoir un bouchon supplémentaire pour le tube du dispositif de stockage. Un simple tube, sans tête de tube et sans élément lesté, éventuellement fermé par un opercule, peut également être utilisé selon différents modes de réalisation de l'invention.

Selon encore d'autres modes de réalisation, la partie mâle peut présenter une forme différente de celle illustrée en figures 2 à 5. En particulier, le fût mâle 31 et sa tête 32 peuvent présenter un diamètre sensiblement identique. On assure de cette façon une découpe propre de l'oreille, en limitant les risques de déchirures. Selon d'autres modes de réalisation, il est également possible d'utiliser une aiguille de biopsie, coulissant à l'intérieur du fût mâle, plutôt qu'un emporte-pièce fixé à l'extrémité du fût mâle.

L'invention permet ainsi de réaliser des opérations de marquage et de prélèvement optimisées, de façon simple et rapide pour l'utilisateur, qui n'a pas à réaliser lui-même plusieurs actions pour perforer le tissu animal, pousser l'échantillon dans le dispositif de stockage, fermer le dispositif de stockage, fermer la tête de la partie femelle, etc, toutes ces opérations pouvant avantageusement être réalisées à partir d'une seule action sur l'outil de pose (par exemple une action manuelle, électrique, pneumatique ou autre sur les poignées de l'outil).

## Revendications

1. Système d'identification animale comprenant :
- une partie mâle (30), coopérant avec au moins un élément de coupe destiné à découper un échantillon de tissu animal,
- une partie femelle (40) à tête ouverte (42), présentant un canal traversant, et
- un dispositif amovible de stockage (50) dudit échantillon, destiné à être fixé à une extrémité (422) dudit canal traversant,
**caractérisé en ce que** ledit système comprend également des moyens de fermeture de ladite tête (42) aptes à prendre au moins deux positions, dont une position d'ouverture antérieure à un prélèvement, dans laquelle ledit canal est ouvert de part en part, permettant le passage dudit échantillon au travers dudit canal traversant, et une position de fermeture irréversible postérieure au prélèvement, dans laquelle ledit canal est fermé au voisinage de ladite extrémité (422).

2. Système d'identification animale selon la revendication 1, **caractérisé en ce que** lesdits moyens de fermeture comprennent un bouchon (35)

3. Système d'identification animale selon la revendication 2, **caractérisé en ce que** ladite partie femelle comprend des moyens de retenue (43) dudit bouchon dans ladite position de fermeture.

4. Système d'identification animale selon la revendication 3, **caractérisé en ce que** lesdits moyens de retenue comprennent une rondelle présentant au moins une lame ressort.

5. Système d'identification animale selon la revendication 3, **caractérisé en ce que** lesdits moyens de retenue comprennent au moins un pêne (441) pouvant prendre au moins deux positions, dont une position de déverrouillage dans laquelle ledit au moins un pêne est maintenu en position par ledit dispositif de stockage, et une position de verrouillage dans laquelle ledit au moins un pêne est libéré et retient ledit bouchon.

6. Système d'identification animale selon la revendication 5, **caractérisé en ce que** lesdits moyens de retenue comprennent également au moins un élément de blocage (443) permettant de bloquer ledit moins un pêne dans ladite position de verrouillage.

7. Système d'identification animale selon la revendication 6, **caractérisé en ce que** ledit au moins un élément de blocage est une bille.

8. Système d'identification animale selon la revendication 3, **caractérisé en ce que** lesdits moyens de retenue comprennent une pièce formant ressort, comprenant au moins deux mâchoires mobiles telles que dans ladite position d'ouverture, lesdites mâchoires sont contraintes en ouverture et maintiennent ledit dispositif de stockage, et dans ladite position de fermeture, lesdites mâchoires sont libérées et verrouillent ledit bouchon.

9. Système d'identification animale selon la revendication 4, la revendication 5 ou la revendication 8, **caractérisé en ce que** ledit bouchon comprend au moins une gorge, destinée à recevoir une extrémité libre de ladite au moins une lame ressort, ledit au moins un pêne ou lesdites mâchoires dans ladite position de fermeture.

10. Système d'identification animale selon l'une quelconque des revendications 2 à 9, **caractérisé en ce que** ledit bouchon présente un premier diamètre dans ladite position d'ouverture, et un deuxième diamètre, supérieur audit premier diamètre, dans ladite position de fermeture.

11. Système d'identification animale selon l'une quelconque des revendications 2 à 10, **caractérisé en ce que** ledit bouchon comprend une substance spécifique, libérée dans ladite position de fermeture, ladite substance spécifique appartenant au groupe comprenant :
- une colle ;
- une résine.

12. Système d'identification animale selon l'une quelconque des revendications 2 à 11, **caractérisé en ce que** ledit bouchon est présent dans un fût (31) de ladite partie mâle dans ladite position d'ouverture.

13. Système d'identification animale selon la revendication 1, **caractérisé en ce que** lesdits moyens de fermeture comprennent au moins un obturateur (81) mobile entre lesdites positions d'ouverture et de fermeture,
**en ce que**, dans ladite position d'ouverture, ledit au moins un obturateur est maintenu en position par ledit dispositif de stockage,
et **en ce que**, dans ladite position de fermeture, ledit au moins un obturateur obture ledit canal au voisinage de ladite extrémité.

14. Système d'identification animale selon la revendication 13, **caractérisé en ce que** lesdits moyens de fermeture comprennent une pièce formant ressort comprenant deux obturateurs mobiles, appelés mâchoires, tels que dans ladite position d'ouverture, lesdites mâchoires sont contraintes en ouverture et maintiennent ledit dispositif de stockage, et dans ladite position de fermeture, lesdites mâchoires sont libérées et obturent le canal au voisinage de ladite extrémité.

15. Système d'identification animale selon l'une quelconque des revendications 1 à 14, **caractérisé en ce qu'**il comprend un élément de renfort, inséré dans un fût de ladite partie mâle, ou dans ledit fût selon la revendication 12, destiné à renforcer la rigidité dudit fût lors du prélèvement.

## Patentansprüche

1. System zur Kennzeichnung von Tieren, umfassend:
- ein männliches Teil (30), das mit mindestens einem Schneideelement zusammenwirkt, welches dazu bestimmt ist, eine Probe von tierischem Gewebe herauszuschneiden,
- ein weibliches Teil (40) mit offenem Kopfende (42), das einen durchgehenden Kanal aufweist, und
- eine austauschbare Speichervorrichtung (50) für die Probe, die dazu bestimmt ist, an einem Ende (422) des durchgehenden Kanals befestigt zu sein,
**dadurch gekennzeichnet, dass** das System auch Verschlussmittel des Kopfendes (42) umfasst, die dazu geeignet sind, mindestens zwei Positionen einzunehmen, mit einer Öffnungsposition vor einer Probenentnahme, in welcher der Kanal vollständig geöffnet ist, sodass der Durchgang für die Probe durch den durchgehenden Kanal ermöglicht wird, und mit einer nicht umkehrbaren Verschlussposition nach der Probenentnahme, in welcher der Kanal in der Nähe des Endes (422) geschlossen ist.

2. System zur Kennzeichnung von Tieren nach Anspruch 1, **dadurch gekennzeichnet, dass** die Verschlussmittel einen Stopfen (35) umfassen.

3. System zur Kennzeichnung von Tieren nach Anspruch 2, **dadurch gekennzeichnet, dass** das weibliche Teil Haltemittel (43) des Stopfens in der Verschlussposition umfasst.

4. System zur Kennzeichnung von Tieren nach Anspruch 3, **dadurch gekennzeichnet, dass** die Haltemittel eine Scheibe umfassen, die mindestens eine Blattfeder aufweist.

5. System zur Kennzeichnung von Tieren nach Anspruch 3, **dadurch gekennzeichnet, dass** die Haltemittel mindestens einen Riegel (441) umfassen, der mindestens zwei Positionen einnehmen kann, mit einer Freigabeposition, in welcher der mindestens eine Riegel durch die Speichervorrichtung in Position gehalten wird, und mit einer Verriegelungsposition, in welcher der mindestens eine Riegel freigegeben wird und den Stopfen zurückhält.

6. System zur Kennzeichnung von Tieren nach Anspruch 5, **dadurch gekennzeichnet, dass** die Haltemittel auch mindestens ein Blockierelement (443) umfassen, das es ermöglicht, den mindestens einen Riegel in der Sperrposition zu blockieren.

7. System zur Kennzeichnung von Tieren nach Anspruch 6, **dadurch gekennzeichnet, dass** das mindestens eine Blockierelement eine Kugel ist.

8. System zur Kennzeichnung von Tieren nach Anspruch 3, **dadurch gekennzeichnet, dass** die Haltemittel ein Federstück umfassen, das mindestens zwei Backen umfasst, die derart beweglich sind, dass die Backen in der Öffnungsposition offengehalten sind und die Speichervorrichtung halten, und dass in der Verschlussposition die Backen freigegeben sind und den Stopfen verriegeln.

9. System zur Kennzeichnung von Tieren nach Anspruch 4, Anspruch 5 oder Anspruch 8, **dadurch gekennzeichnet, dass** der Stopfen eine Einschnürung umfasst, die dazu bestimmt ist, ein freies Ende der zumindest einen Blattfeder, des zumindest einen Riegels oder der Backen in der Verschlussposition aufzunehmen.

10. System zur Kennzeichnung von Tieren nach einem der Ansprüche 2 bis 9, **dadurch gekennzeichnet, dass** der Stopfen einen ersten Durchmesser in der Öffnungsposition, und einen zweiten Durchmesser in der Verschlussposition, der größer ist als der erste Durchmesser, aufweist.

11. System zur Kennzeichnung von Tieren nach einem der Ansprüche 2 bis 10, **dadurch gekennzeichnet, dass** der Stopfen eine spezifische Substanz umfasst, die in der Verschlussposition freigegeben wird, wobei die spezifische Substanz zu einer Gruppe gehört, die umfasst:
- einen Klebstoff;
- ein Harz.

12. System zur Kennzeichnung von Tieren nach einem der Ansprüche 2 bis 11, **dadurch gekennzeichnet, dass** der Stopfen in einem Schaft (31) des männlichen Teils in der Öffnungsposition angeordnet ist.

13. System zur Kennzeichnung von Tieren nach Anspruch 1, **dadurch gekennzeichnet, dass** die Verschlussmittel mindestens ein Verschlussstück (81) umfassen, welches zwischen der Öffnungsposition und der Verschlussposition bewegbar ist,
dadurch, dass in der Öffnungsposition das zumindest eine Verschlussstück durch die Speichervorrichtung in Position gehalten wird,
und dadurch, dass in der Verschlussposition das zumindest eine Verschlussstück den Kanal in der Nähe des Endes verschließt.

14. System zur Kennzeichnung von Tieren nach Anspruch 13, **dadurch gekennzeichnet, dass** die Verschlussmittel ein Federstück umfassen, das zwei bewegliche Verschlussstücke, genannt Backen, derart umfasst, dass die Backen in der Öffnungsposition offengehalten sind und die Speichervorrichtung halten, und dass die Backen in der Verschlussposition freigegeben sind und den Kanal in der Nähe des Endes verschließen.

15. System zur Kennzeichnung von Tieren nach einem der Ansprüche 1 bis 14, **dadurch gekennzeichnet, dass** es ein Verstärkungselement umfasst, dass in einen Schaft des männlichen Teils oder in den Schaft nach Anspruch 12 eingeführt wurde, das dazu bestimmt ist, die Steifigkeit des Schaftes bei der Probenentnahme zu verstärken.

## Claims

1. System for identifying an animal comprising:
- a male part (30), cooperating with at least one cutting element intended to cut out a sample of animal tissue,
- a female part (40) with open head (42) having a through-channel, and
- a detachable device (50) for storing said sample, intended to be fixed to one end (422) of said through-channel,
wherein said system also comprises means for closing said head (42) configured to take at least two positions, including an open position prior to a collecting, in which said channel is open from one side to the other, enabling the passage of said sample through the through-channel, and an irreversible closed position after the collecting operation, in which said channel is closed in the vicinity of said end (422).

2. System for identifying an animal according to claim 1, wherein said means for closing comprise a plug (35).

3. System for identifying an animal according to claim 2, wherein said female part comprises means (43) for retaining said plug in said closed position.

4. System for identifying an animal according to claim 3, wherein said retaining means comprise a washer having at least one spring leaf.

5. System for identifying an animal according to claim 3, wherein said retaining means comprise at least one bolt (441) which can take at least two positions, of which one is an unlocked position in which said at least one bolt is held in position by said storage device and one is a locked position in which said at least one bolt is released and retains said plug.

6. System for identifying an animal according to claim 5, wherein said retaining means also comprise at least one blocking element (443) enabling said at least one bolt to be blocked in said locked position.

7. System for identifying an animal according to claim 6, wherein said at least one blocking element is a ball.

8. System for identifying an animal according to claim 3, wherein said retaining means comprise a spring-forming part comprising at least two mobile jaws such that, in said open position, said jaws are stressed in opening and hold said storage device and, in said closed position, said jaws are released and lock said plug.

9. System for identifying an animal according to claim 4, claim 5 or claim 8, wherein said plug comprises at least one groove, intended to receive a free end of said at least one spring leaf or said at least one bolt or said jaws in said closed position.

10. System for identifying an animal according to any one of the claims 2 to 9, wherein said plug has a first diameter in said open position and a second diameter, greater than said first diameter, in said closed position.

11. System for identifying an animal according to any one of the claims 2 to 10, wherein said plug comprises a specific substance released in said closed position, said specific substance belonging to the group comprising:
- a glue;.
- a resin.

12. System for identifying an animal according to any one of the claims 2 to 11, wherein said plug is present in a pin (31) of said male part in said open position.

13. System for identifying an animal according to claim 1, wherein said means for closing comprise at least one shutter (81) that is mobile between said open and closed positions, wherein, in said open position, said at least one shutter is held in position by said storage device, and wherein, in said closed position, said at least one shutter shuts off said channel in the vicinity of said end.

14. System for identifying according to claim 13, wherein said the means for closing comprise a spring-forming part comprising two shutters, called jaws, such that, in said open position, said jaws are stressed in opening and hold said storage device and, in said closed position, said jaws are released and shut off the channel in the vicinity of said end.

15. System for identifying an animal according to any one of the claims 1 to 14, wherein it comprises a reinforcement element inserted into a pin of said male part, or into said pin according to claim 12, intended to reinforce the rigidity of said pin during the collecting operation.
